# EUROPÄISCHE PATENTSCHRIFT

(11) **EP 2 753 338 B1**
(45) Veröffentlichungstag und Bekanntmachung des Hinweises auf die Patenterteilung: **13.07.2016**
(21) Anmeldenummer: 12758407.6
(22) Anmeldetag: 05.09.2012
(51) Int. Cl.: A61K 31/724, A61K 31/718, A61P 1/16, A61P 7/08, A61P 43/00

(54) **PHARMAZEUTISCHE ZUSAMMENSETZUNGEN ENTHALTEND CARBOXYLIERTE STÄRKE**
PHARMACEUTICAL COMPOSITIONS CONTAINING CARBOXYLATED STARCH
COMPOSITIONS PHARMACEUTIQUES COMPRENANT DE L'AMIDON CARBOXYLÉ

(30) Priorität: 07.09.2011 DE 102011112526; 07.09.2011 US 201161531828 P
(43) Veröffentlichungstag der Anmeldung: 16.07.2014
(73) Patentinhaber: Fresenius Medical Care Deutschland GmbH, 61352 Bad Homburg (DE)
(72) Erfinder: FENN, Dominik, 63225 Langen (DE); SCHWEITZER, Thomas, 66589 Wemmetsweiler (DE)
(74) Vertreter: Breuninger, Marcus
(86) Internationale Anmeldenummer: PCT/EP2012/003726
(87) Internationale Veröffentlichungsnummer: WO 2013/034292

(56) Entgegenhaltungen:
- WO-A1-00/33851
- WO-A1-03/035699
- JP-A- 8 071 146
- JUYOUNG YOON ET AL: "A General Method for the Synthesis of Cyclodextrinyl Aldehydes and Carboxylic Acids", THE JOURNAL OF ORGANIC CHEMISTRY, Bd. 60, Nr. 9, 1. Mai 1995 (1995-05-01), Seiten 2792-2795, XP055044064, ISSN: 0022-3263, DOI: 10.1021/jo00114a030
- YASUHISA KURODA ET AL: "5A,5D-dicarboxy-[beta]-cyclodextrin derivatives - a route for regioselectively difunctionalized permethyl-[beta]-cyclodextrin", TETRAHEDRON LETTERS, Bd. 30, Nr. 51, 1. Januar 1989 (1989-01-01), Seiten 7225-7228, XP055044066, ISSN: 0040-4039, DOI: 10.1016/S0040-4039(01)93941-3
- ROUSSEAU C ET AL: "An artificial enzyme that catalyzes hydrolysis of aryl glycosides", TETRAHEDRON LETTERS, ELSEVIER, AMSTERDAM, NL, Bd. 45, Nr. 47, 15. November 2004 (2004-11-15), Seiten 8709-8711, XP027280360, ISSN: 0040-4039 [gefunden am 2004-10-22]
- CYRIL ROUSSEAU ET AL: "Artificial Glycosyl Phosphorylases", CHEMISTRY - A EUROPEAN JOURNAL, Bd. 11, Nr. 17, 19. August 2005 (2005-08-19), Seiten 5094-5101, XP055044060, ISSN: 0947-6539, DOI: 10.1002/chem.200500364
- BARBIER M; BRETON, T; SERVAT, K; GRAND, E; KOKOH, B; KOVENSKY, J: "Selective TEMPO-Catalyzed Chemicals vs. Electrochemical Oxidation of Carbohydrate Derivatives", JOURNAL OF CARBOHYDRATE CHEMISTRY, Bd. 25, 10. Februar 2006 (2006-02-10), Seiten 253-266, XP9164623, ISSN: 0732-8303, DOI: 10.1080/07328300600636819
- HEINZE T ET AL: "NEW POLYMERS BASED ON CELLULOSE", LENZINGER BERICHTE, LENZING AG, LENZING, AT, Bd. 79, 1. Januar 2000 (2000-01-01), Seiten 39-44, XP001086679, ISSN: 0024-0907 in der Anmeldung erwähnt

## Beschreibung

Die vorliegende Erfindung betrifft pharmazeutische Zusammensetzungen enthaltend carboxylierte Stärke sowie deren Verwendung als Osmotika, insbesondere zur Verwendung in der Behandlung von chronischem Nierenversagen mittels Dialyse.

Osmotisch aktive Verbindungen (Osmotika) finden in der Pharmazie und Medizin breite Anwendung. So werden Osmotika beispielsweise zur Einstellung der Tonizität von Arzneimitteln, insbesondere parenteralen Medikamenten verwendet. Hierbei wird der osmotische Druck eines Arzneimittels je nach Art der Anwendung hypotonisch, hypertonisch oder isotonisch eingestellt. Beispielsweise kann der osmotische Druck einer parenteralen Arzneilösung dem osmotischen Druck des menschlichen Bluts durch Zugabe eines Osmotikums angeglichen werden (isoosmotische Lösungen).

Ferner werden Osmotika bei der Behandlung von chronischem Nierenversagen mittels Dialyse, insbesondere bei der Hämo- oder Peritonealdialyse, eingesetzt um dem Dialysepatienten überschüssiges Wasser zu entziehen.

Das Peritonealdialyseverfahren beruht darauf, dass eine Lösung, die osmotisch aktive Verbindungen enthält, über einen Katheter in die Bauchhöhle des Dialysepatienten eingebracht wird. Diese Lösung wird für eine bestimmte Zeit (üblicherweise einige Stunden) in der Bauchhöhle des Patienten belassen und entfaltet dort ihre osmotische Wirkung; d.h. dem Patienten wird körpereigenes Wasser in die Bauchhöhle entzogen. Nach einer bestimmten Verweildauer wird die nunmehr verdünnte Peritonealdialyselösung über einen Katheter abgelassen.

Dieses Prinzip findet in verschiedenen Verfahren der Peritonealdialysebehandlung Anwendung. Nach Bedarf können beispielsweise die Verfahren der intermittierenden (IPD), nächtlichen intermittierenden (NIPD), kontinuierlichen zyklischen (CCPD) oder kontinuierlichen ambulanten Peritonealdialyse (CAPD), angewendet werden. Bei IPD, NIPD und CCPD kommen Geräte zum Einsatz, die den Patienten bei der Durchführung des Peritonealdialyseverfahrens unterstützen. Die CAPD ist ein manuelles Verfahren.

Durch die Zugabe von osmotisch aktiven Verbindungen soll insbesondere gewährleistet werden, dass der osmotische Druck der Peritonealdialyselösung während der gesamten Verweildauer in der Bauchhöhle hoch genug ist, um dem Patienten Wasser zu entziehen; d.h. Wasser geht aus dem Kreislauf des Patienten in die Bauchhöhle über (Ultrafiltration).

Jedoch kommt es aufgrund des Wasserübertritts in die Bauchhöhle zwangsläufig zu einer Verdünnung der eingebrachten Peritonealdialyselösung. Diese Verdünnung hat zur Folge, dass die Konzentration der osmotisch aktiven Verbindung und somit auch der osmotische Druck dieser Lösung abnehmen.

Nimmt der osmotische Druck der Peritonealdialyselösung aufgrund dieser Verdünnung ab, so hat dies wiederum zur Folge, dass auch der pro Zeiteinheit stattfindende Wasserübertritt in die Bauchhöhle abnimmt oder möglicherweise gänzlich zum Erliegen kommt. In diesen Fällen findet also mit fortschreitender Verweildauer der Peritonealdialyselösung in der Bauchhöhle des Patienten kein effektiver Wasserentzug mehr statt.

Durch Absorption von osmotisch aktiven Verbindungen in den Blutkreislauf des Patienten, kann sich die Richtung des Wasserübertritts sogar umkehren, d.h. Wasser geht aus der Bauchhöhle in den Blutkreislauf des Patienten über (negative Ultrafiltration). Dies ist dann der Fall, wenn die verdünnte Peritonealdialyselösung in der Bauchhöhle einen geringeren osmotischen Druck als das körpereigene Wasser (z.B. das Blut) des Patienten aufweist.

Durch Zugabe geeigneter osmotisch aktiver Verbindungen zur Peritonealdialyselösung kann der osmotische Druck über eine für die Peritonealdialyse geeignete Behandlungsdauer aufrechterhalten werden, so dass es innerhalb der Verweildauer der Lösung in der Bauchhöhle zu keinem übermäßigen Rückgang der Ultrafiltration kommt. Somit wird auch eine negative Ultrafiltration weitestgehend verhindert.

Die in der Peritonealdialysebehandlung eingesetzten Lösungen enthalten in der Regel Zuckermonomere oder -polymere, wie beispielsweise Glucose oder Polyglucose (z.B. Stärkederivate), als osmotisch wirksame Verbindungen.

US 4,761,237 offenbart eine Peritonealdialyselösung enthaltend Stärkehydrolysat-Glucosepolymere mit einem durchschnittlichen Polymerisationsgrad von mindestens 4.

JP 8071146 betrifft eine Peritonealdialyselösung, die α- oder γ-Cyclodextrin, 2-Hydroxyethylether-, 2-Hydroxypropylether-, 6-O-a-Glucosyl- oder 6-O-α-Maltosylderivate von α-, β- und γ-Cyclodextrin enthält.

Yoon et al. (J. Org. Chem. 1995, 60, 2792-2795) und Kuroda et al. (Tetrahedron Lett., 1989, 30 (51), 7225-7228) offenbaren Cyclodextrinderivate.

Rousseau et al. offenbaren in Tetrahedron Lett., 45 (2004) 8709-8711 und Chem. Eur. J. 2005, 11, 5094-5101 künstliche Emzyme auf Basis von Cyclodextrin-derivaten.

EP 1 051 183 offenbart die Verwendung eines Glucosepolymers bestehend aus D-Sorbitol und D-Glucit bzw. Gluconsäure zur Verwendung in der Peritonealdialyse.

US 4,339,433 offenbart die Verwendung von Carboxymethylpolysacchariden als Osmotikum.

WO 86/00228 betrifft die Verwendung von Polyanionen und -kationen zur Verwendung in der Hämo- und Peritonealdialyse.

Die vorliegende Erfindung betrifft carboxylierte Stärke und carboxylierte Stärkederivate der allgemeinen Formel I, wobei n und m Ganzzahlen und größer oder gleich 1 sind und X entweder H oder eine über eine α-1,6-glykosidische Bindung verbundene Glucoseeinheit ist.

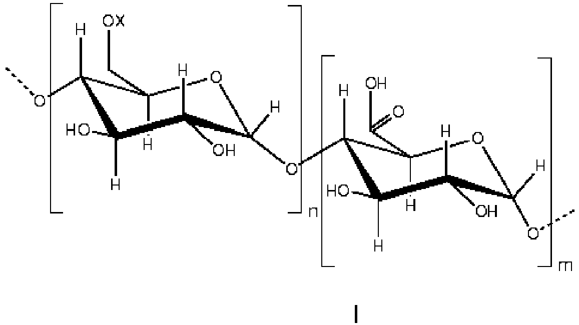

Stärke besteht aus D-Glucose-Einheiten, die über glykosidische Bindungen miteinander verknüpft sind. Stärke enthält zu etwa 20-30% Amylose und zu etwa 70-80% Amylopektin. Amylose besteht aus linearen Ketten mit helikaler Struktur, die nur α-1,4-glykosidisch verknüpft sind. Amylopektin besteht aus verzweigten Strukturen mit α-1,4- und α-1,6-glykosidischen Verknüpfungen. Nach etwa 30 α-1,4-glykosidischen Verknüpfungen kommt es zu einer Verzweigung über eine α-1,6-glykosidische Bindung. Stärke stellt also ein überwiegend über α-1,4-glykosidischen Verknüpfungen verbundenes Polymer dar. Die Hydroxylgruppen an Position C-6 sind damit zum überwiegenden Teil nicht an α-1,6-glykosidischen Bindungen beteiligt und können carboxyliert werden. "Überwiegend" bedeutet in diesem Zusammenhang mindestens 85%. Dies bedeutet, dass mindestens 85% der Glucoseeinheiten ausschließlich über α-1,4-glykosidischen Verknüpfungen miteinander verbunden sind.

Ein Beispiel für ein Stärkederivat ist Icodextrin. Icodextrin ist ein aus Stärke erhältliches, wasserlösliches Glucosepolymer, bei dem die Glucoseeinheiten überwiegend über α-1,4-glykosidischen und zu weniger als 10% über α-1,6-glykosidische Bindungen miteinander verknüpft sind. Icodextrin hat typischerweise eine mittlere Molmasse von ca. 13 000 bis 19 000 Dalton (bezogen auf das Massenmittel, Mw) bzw. ca. 5 000 bis 6 500 Dalton (bezogen auf das Zahlenmittel, Mn).

Die Variablen m und n der allgemeinen Formel I sind Ganzzahlen größer oder gleich 1. Die Summe aus m+n ist damit größer oder gleich 2. In einer bevorzugten Ausführungsform ist die Summe aus m+n bevorzugt größer 20, besonders bevorzugt 20 bis 250, insbesondere 25 bis 150.

Die mittlere Molmasse (bezogen auf das Massenmittel, Mw) der erfindungsgemäßen carboxylierten Stärke beträgt 5 bis 30 kD. In einer bevorzugten Ausführungsform ist die mittlere Molmasse (Mw) bevorzugt 10 bis 20 kD, insbesondere 13 bis 19 kD.

Die mittlere Molmasse (bezogen auf das Zahlenmittel, Mn) der erfindungsgemäßen carboxylierten Stärke beträgt 2 bis 12 kD. In einer bevorzugten Ausführungsform ist die mittlere Molmasse (Mn) bevorzugt 4 bis 8 kD, insbesondere 5 bis 7 kD.

Carboxylierte Stärke ist beispielsweise durch ein Verfahren erhältlich, wie es von T. Heinze *et al.* zur Herstellung von carboxylierter Cellulose veröffentlicht worden ist. (T. Heinze, M. Vieira, U. Heinze; New polymers based on cellulose; Lenzinger Berichte 79 (2000) 39-44).

Eine Aufgabe der vorliegenden Erfindung ist es, Stärkederivate zur Verfügung zu stellen, die eine hohe Wasserlöslichkeit, eine verbesserte osmotische Wirksamkeit und eine erhöhte Ultrafiltration gegenüber den Stärkederivaten des Standes der Technik aufweisen, und die somit für pharmazeutische Zusammensetzungen insbesondere zur Dialysebehandlung geeignet sind.

Diese Aufgabe wird durch den Gegenstand der Patentansprüche gelöst.

Die erfindungsgemäßen carboxylierten Stärkederivate zeichnen sich gegenüber den Stärkederivaten des Standes der Technik durch ein einheitlicheres Substitutionsmuster aus, d.h. sie sind in ihrer Struktur genauer definiert (z.B. geringerer Anteil unterschiedlicher Stellungsisomere; einheitlicher Oxidationsgrad).

Dies hat zur Folge, dass unerwünschte Nebenwirkungen, die auf dem Vorhandensein verschiedenster unterschiedlicher Substitutionsmuster beruhen, weitestgehend ausgeschlossen werden. Dies erhöht die Sicherheit für den Patienten.

Insbesondere kann auch die Wirksamkeit der erfindungsgemäßen carboxylierten Stärkederivate auf definierte Verbindungen zurückgeführt werden und beruht nicht auf den Wirkungen eines komplexen Gemisches unterschiedlichster Stärkederivate. Auch dies erhöht die Sicherheit für den Patienten und erleichtert insbesondere die Evaluierung pharmakologischer bzw. klinischer Daten.

Zudem können Gemische an carboxylierten Stärkederivaten, die nur eine eingeschränkt reproduzierbare Zusammensetzung aufweisen, zu mangelnder Reproduzierbarkeit bei der Ermittlung experimenteller Daten führen, wie beispielsweise bei der Durchführung pharmakologischer oder toxikologischer *in vivo-* oder *ex vivo*-Experimenten sowie bei der Durchführung klinischer Studien.

Die sauren Reste -COOH können deprotoniert, d.h. anionisch vorliegen und mit Kationen - beispielsweise Natrium, Kalium, Magnesium, Calcium, Ammonium - als Salz vorliegen.

Im Sinne dieser Beschreibung steht der Begriff "Oxidationsgrad" für die durchschnittliche Molzahl des Restes -COOH bezogen auf 1 Mol Glucoseeinheiten, die nicht über eine α-1,6-glykosidische Bindung mit einer weiteren Glucoseeinheit verbunden ist. Der Oxidationsgrad kann Werte zwischen ≥0 und ≤1 annehmen. Ein Oxidationsgrad von 0 entspricht nicht oxidierter Stärke und ein Oxidationsgrad von 1 bedeutet, dass jede Glucoseeinheit in Position 6 oxidiert ist.

In einer bevorzugten Ausführungsform ist der Oxidationsgrad der erfindungsgemäßen carboxylierten Stärke ≥0,01 und ≤1, bevorzugt zwischen ≥0,05 und ≤0,98, besonders bevorzugt zwischen ≥0,1 und ≤0,95.

Vorzugsweise weist eine 7,5 gewichtsprozentige wässrige Lösung der erfindungsgemäßen carboxylierten Stärkederivate eine theoretische Osmolarität (bezogen auf das Zahlenmittel der Molmasse, Mn) von ≥5 mosm/L, bevorzugter größer als ≥7,5 mosm/L, noch bevorzugter größer als ≥10 mosm/L, am Bevorzugtesten größer als ≥12,5 mosm/L und insbesondere größer als ≥15 mosm/L auf.

Zum Zwecke dieser Beschreibung steht der Ausdruck "theoretische Osmolarität" für die theoretisch berechnete Osmolarität. Methoden zur Berechnung dieses Werts sind dem Fachmann bekannt.

In einer bevorzugten Ausführungsform beträgt der kolloidosmotische Druck einer 7,5 gewichtsprozentigen Lösung der erfindungsgemäßen carboxylierten Stärkederivate ≥50 mosm/L oder ≥60 mosm/L, bevorzugter ≥70 mosm/L oder ≥80 mosm/L, noch bevorzugter ≥90 mosm/L oder ≥100 mosm/L, am Bevorzugtesten ≥110 mosm/L oder ≥120 mosm/L und insbesondere ≥130 mosm/L oder ≥140 mosm/L.

In einer weiteren bevorzugten Ausführungsform beträgt der kolloidosmotische Druck einer 7,5 gewichtsprozentigen Lösung der erfindungsgemäßen carboxylierten Stärkederivate ≥150 mosm/L oder ≥160 mosm/L, bevorzugter ≥170 mosm/L oder ≥180 mosm/L, noch bevorzugter ≥190 mosm/L oder ≥200 mosm/L, am Bevorzugtesten ≥210 mosm/L oder ≥220 mosm/L und insbesondere ≥230 mosm/L oder ≥240 mosm/L.

In einer weiteren bevorzugten Ausführungsform beträgt der kolloidosmotische Druck einer 7,5 gewichtsprozentigen Lösung der erfindungsgemäßen carboxylierten Stärkederivate 50 bis 500 mosm/L, bevorzugter 75 mosm/L bis 400 mosm/L, noch bevorzugter 100 bis 300 mosm/L, am Bevorzugtesten 110 mosm/L bis 275 mosm/L und insbesondere 120 mosm/L bis 250 mosm/L.

In einer weiteren bevorzugten Ausführungsform beträgt der kolloidosmotische Druck einer 7,5 gewichtsprozentigen Lösung der erfindungsgemäßen carboxylierten Stärkederivate 100 bis 500 mosm/L, bevorzugter 100 mosm/L bis 400 mosm/L, noch bevorzugter 100 bis 350 mosm/L, am Bevorzugtesten 100 mosm/L bis 325 mosm/L und insbesondere 100 mosm/L bis 290 mosm/L.

Zum Zwecke dieser Beschreibung steht der Ausdruck "kolloidosmotischer Druck" für den experimentell gemessenen osmotischen Druck der Lösung, der sich aus dem osmotischen und onkotischen Druck zusammensetzt. Geeignete Methoden zur experimentellen Bestimmung dieses Werts sind dem Fachmann bekannt.

Die Osmolalität einer 7,5 gewichtsprozentigen wässrigen Lösung der erfindungsgemäßen carboxylierten Stärke beträgt vorzugsweise >5 mosm/kg, bevorzugter ≥7,5 mosm/kg, noch bevorzugter ≥10 mosm/kg, am Bevorzugtesten ≥12 mosm/kg und insbesondere ≥15 mosm/kg.

Zum Zwecke dieser Beschreibung steht der Begriff "Osmolalität" für die mittels Gefrierpunktserniedrigung experimentell bestimmten Osmolalität der Lösung. Methoden zur Bestimmung der Gefrierpunktserniedrigung sind dem Fachmann bekannt.

Die mittels Gefrierpunkterniedrigung experimentell bestimmte Osmolarität einer 7,5 gewichtsprozentigen wässrigen Lösung der erfindungsgemäßen carboxylierten Stärkederivate beträgt vorzugsweise ≥15 mosm/L, bevorzugter ≥17 mosm/L, noch bevorzugter ≥19 mosm/L, am Bevorzugtesten ≥21 mosm/L und insbesondere ≥23 mosm/L.

In einer bevorzugten Ausführungsform finden die erfindungsgemäßen carboxylierten Stärkederivate Verwendung in der Dialysebehandlung, vorzugsweise in der Hämo- und/oder Peritonealdialysebehandlung.

Die erfindungsgemäßen carboxylierten Stärkederivate sind insbesondere zur Verwendung in der Peritonealdialysebehandlung geeignet.

Ein weiterer Gegenstand dieser Erfindung betrifft Dialyselösungen enthaltend wenigstens eine erfindungsgemäße carboxylierte Stärke oder ein erfindungsgemäßes carboxyliertes Stärkederivat.

In einer bevorzugten Ausführungsform ist die erfindungsgemäße Dialyselösung eine Hämodialyselösung oder eine Peritonealdialyselösung. Die erfindungsgemäße Dialyselösung ist insbesondere eine Peritonealdialyselösung.

Darreichungsformen, die in der Dialysebehandlung eingesetzt werden, sind vorzugsweise Konzentrate in Mehrkomponenten-Systemen oder gebrauchsfertige Dialyselösungen.

Für die Zwecke dieser Erfindung umfasst der Ausdruck "Dialyselösung" eine gebrauchsfertige Darreichungsform zur Dialysebehandlung, d.h. eine flüssige Zubereitung, die als solche zur Applikation geeignet ist. Insbesondere muss die Dialyselösung vor der Applikation nicht verdünnt und/ oder mit anderen Zubereitungen gemischt werden.

Im Gegensatz zu den vorstehend beschriebenen Dialyselösungen werden Konzentrate, die entweder in flüssiger, halbfester oder fester Form vorliegen können, vor der Applikation mit Wasser oder wässrigen Lösungen verdünnt oder in Wasser oder wässrigen Lösungen gelöst. In analoger Weise müssen die Komponenten eines Mehrkomponenten-Systems vor der Applikation miteinander gemischt werden um eine gebrauchsfertige Dialyselösung zu erhalten. Konzentrate und Mehrkomponenten-Systeme können also als Vorstufe der erfindungsgemäßen Dialyselösung angesehen werden.

Die erfindungsgemäße Dialyselösung ist vorzugsweise eine Hämodialyse- oder eine Peritonealdialyselösung. Hämodialyse- und Peritonealdialyselösungen enthalten üblicherweise Elektrolyte in einer Konzentration, die im Wesentlichen der Plasma-Elektrolyt-Konzentration entspricht. Elektrolyte umfassen üblicherweise Natrium-, Kalium-, Calcium-, Magnesium- und Chlorid-Ionen.

Dialyselösungen haben üblicherweise einen physiologisch verträglichen pH-Wert. Dies wird vorzugsweise erreicht durch Puffer (Puffer-Systeme), die selbst zum Gesamtgehalt an Elektrolyten beitragen können. Die Puffer sind vorzugsweise Hydrogencarbonat, Lactat oder Pyruvat.

Ferner besitzen Dialyselösungen üblicherweise eine physiologisch verträgliche Osmolarität. Dies wird in der Regel erreicht durch die in der Dialyselösung enthaltenen Elektrolyte und erfindungsgemäßen carboxylierten Stärkederivate, die als osmotisch aktive Verbindungen (Osmotika) in der gewünschten Konzentration physiologisch verträglich sind.

Die erfindungsgemäße Dialyselösung besitzt eine Osmolarität im Bereich von vorzugsweise 200 bis 550 mosm/L.

Im Fall, dass die erfindungsgemäße Dialyselösung eine Hämodialyselösung ist, beträgt die Osmolarität vorzugsweise 200 bis 350 mosm/L oder 210 bis 340 mosm/L, bevorzugter 220 bis 330 mosm/L, noch bevorzugter 230 bis 320 mosm/L, am Bevorzugtesten 240 bis 310 mosm/L und insbesondere 250 bis 300 mosm/L. Verfahren zur Messung der Osmolarität und des osmotischen Drucks sind dem Fachmann bekannt. Beispielsweise können diese mit Hilfe eines Membranosmometers oder anderen geeigneten Messmethoden bestimmt werden.

Im Fall, dass die erfindungsgemäße Dialyselösung eine Peritonealdialyselösung ist, beträgt die Osmolarität vorzugsweise 200 bis 570 mosm/L oder 210 bis 560 mosm/L, bevorzugter 220 bis 550 mosm/L, noch bevorzugter 230 bis 540 mosm/L, am Bevorzugtesten 240 bis 530 mosm/L und insbesondere 250 bis 520 mosm/L. In einer bevorzugten Ausführungsform beträgt die Osmolarität 250 ± 50 mosm/L oder 250 ± 45 mosm/L, bevorzugter 250 ± 35 mosm/L, noch bevorzugter 250 ± 25 mosm/L, am Bevorzugtesten 250 ± 15 mosm/L und insbesondere 250 ± 10 mosm/L. In einer weiteren bevorzugten Ausführungsform beträgt die Osmolarität 300 ± 50 mosm/L oder 300 ± 45 mosm/L, bevorzugter 300 ± 35 mosm/L, noch bevorzugter 300 ± 25 mosm/L, am Bevorzugtesten 300 ± 15 mosm/L und insbesondere 300 ± 10 mosm/L. In einer weiteren bevorzugten Ausführungsform beträgt die Osmolarität 350 ± 50 mosm/L oder 350 ± 45 mosm/L, bevorzugter 350 ± 35 mosm/L, noch bevorzugter 350 ± 25 mosm/L, am Bevorzugtesten 350 ± 15 mosm/L und insbesondere 300 ± 10 mosm/L. In einer weiteren bevorzugten Ausführungsform beträgt die Osmolarität 400 ± 50 mosm/L oder 400 ± 45 mosm/L, bevorzugter 400 ± 35 mosm/L, noch bevorzugter 400 ± 25 mosm/L, am Bevorzugtesten 400 ± 15 mosm/L und insbesondere 300 ± 10 mosm/L. In einer weiteren bevorzugten Ausführungsform beträgt die Osmolarität 450 ± 50 mosm/L oder 450 ± 45 mosm/L, bevorzugter 450 ± 35 mosm/L, noch bevorzugter 450 ± 25 mosm/L, am Bevorzugtesten 450 ± 15 mosm/L und insbesondere 450 ± 10 mosm/L. In einer weiteren bevorzugten Ausführungsform beträgt die Osmolarität 500 ± 50 mosm/L oder 500 ± 45 mosm/L, bevorzugter 500 ± 35 mosm/L, noch bevorzugter 500 ± 25 mosm/L, am Bevorzugtesten 500 ± 15 mosm/L und insbesondere 500 ± 10 mosm/L.

Die erfindungsgemäße Dialyselösung hat einen pH-Wert vorzugsweise von 4,0 bis 8,0, bevorzugter von 4,2 bis 7,5, noch bevorzugter von 4,4 bis 6,8, am Bevorzugtesten von 4,6 bis 6,0 oder 4,8 bis 5,5 und insbesondere von 5,0 bis 5,2 oder 5,0±0,1; gemessen bei Raumtemperatur (20 bis 23 °C). In einer bevorzugten Ausführungsform ist der pH-Wert 4,8 ± 1,0 oder 4,8 ± 0.8, bevorzugter 4,8 ± 0,7 oder 4,8 ± 0,6, noch bevorzugter 4,8 ± 0,5 oder 4,8 ± 0,4, am Bevorzugtesten 4,8 ± 0,3 oder 4,8 ± 0,2 und insbesondere 4,8 ± 0,1. In einer weiteren bevorzugten Ausführungsform ist der pH-Wert 5,0 ± 1,0 oder 5,0 ± 0.8, bevorzugter 5,0 ± 0,7 oder 5,0 ± 0,6, noch bevorzugter 5,0 ± 0,5 oder 5,0 ± 0,4, am Bevorzugtesten 5,0 ± 0,3 oder 5,0 ± 0,2 und insbesondere 5,0 ± 0,1. In einer weiteren bevorzugten Ausführungsform ist der pH-Wert 5,2 ± 1,0 oder 5,2 ± 0,8, bevorzugter 5,2 ± 0,7 oder 5,2 ± 0,6, noch bevorzugter 5,2 ± 0,5 oder 5,2 ± 0,4, am Bevorzugtesten 5,2 ± 0,3 oder 5,2 ± 0,2 und insbesondere 5,2 ± 0,1. In einer weiteren bevorzugten Ausführungsform ist der pH-Wert 5,5 ± 1,0 oder 5,5 ± 0,8, bevorzugter 5,5 ± 0,7 oder 5,5 ± 0,6, noch bevorzugter 5,5 ± 0,5 oder 5,5 ± 0,4, am Bevorzugtesten 5,5 ± 0,3 oder 5,5 ± 0,2 und insbesondere 5,5 ± 0,1. In einer weiteren bevorzugten Ausführungsform ist der pH-Wert 6,0 ± 1,0 oder 6,0 ± 0.8, bevorzugter 6,0 ± 0,7 oder 6,0 ± 0,6, noch bevorzugter 6,0 ± 0,5 oder 6,0 ± 0,4, am Bevorzugtesten 6,0 ± 0,3 oder 6,0 ± 0,2 und insbesondere 6,0 ± 0,1. In einer weiteren bevorzugten Ausführungsform ist der pH-Wert 6,5 ± 1,0 oder 6,5 ± 0.8, bevorzugter 6,5 ± 0,7 oder 6,5 ± 0,6, noch bevorzugter 6,5 ± 0,5 oder 6,5 ± 0,4, am Bevorzugtesten 6,5 ± 0,3 oder 6,5 ± 0,2 und insbesondere 6,5 ± 0,1. In einer weiteren bevorzugten Ausführungsform ist der pH-Wert 7,0 ± 1,0 oder 7,0 ± 0.8, bevorzugter 7,0 ± 0,7 oder 7,0 ± 0,6, noch bevorzugter 7,0 ± 0,5 oder 7,0 ± 0,4, am Bevorzugtesten 7,0 ± 0,3 oder 7,0 ± 0,2 und insbesondere 7,0 ± 0,1. In einer weiteren bevorzugten Ausführungsform ist der pH-Wert 7,4 ± 1,0 oder 7,4 ± 0.8, bevorzugter 7,4 ± 0,7 oder 7,4 ± 0,6, noch bevorzugter 7,4 ± 0,5 oder 7,4 ± 0,4, am Bevorzugtesten 7,4 ± 0,3 oder 7,4 ± 0,2 und insbesondere 7,4 ± 0,1. In einer weiteren bevorzugten Ausführungsform ist der pH-Wert 8,0 ± 1,0 oder 8,0 ± 0.8, bevorzugter 8,0 ± 0,7 oder 8,0 ± 0,6, noch bevorzugter 8,0 ± 0,5 oder 8,0 ± 0,4, am Bevorzugtesten 8,0 ± 0,3 oder 8,0 ± 0,2 und insbesondere 8,0 ± 0,1.

Die erfindungsgemäße Dialyselösung enthält ein oder mehrere (z.B. zwei, drei, vier oder fünf) erfindungsgemäße carboxylierte Stärkederivate mit unterschiedlichen Oxidationsgraden; wobei die erfindungsgemäßen carboxylierten Stärkederivate wie oben stehend definiert sind.

Die erfindungsgemäße Dialyselösung enthält erfindungsgemäße carboxylierte Stärkederivate in einer Gesamtkonzentration von vorzugsweise 0,001 mM bis 10 M oder 0,01 bis 1,0 M, bevorzugter 0,10 bis 500 mM, noch bevorzugter 1,0 bis 250 mM, am Bevorzugtesten 10 bis 100 mM und insbesondere 25 bis 90 mM. In einer bevorzugten Ausführungsform ist die Gesamtkonzentration 25 ± 24 mM, bevorzugter 25 ± 20 mM, noch bevorzugter 25 ± 15 mM, am Bevorzugtesten 25 ± 10 mM und insbesondere 25 ± 5 mM. In einer weiteren bevorzugten Ausführungsform ist die Gesamtkonzentration 50 ± 25 mM, bevorzugter 50 ± 20 mM, noch bevorzugter 50 ± 15 mM, am Bevorzugtesten 50 ± 10 mM und insbesondere 50 ± 5 mM. In einer weiteren bevorzugten Ausführungsform ist die Gesamtkonzentration 75 ± 25 mM, bevorzugter 75 ± 20 mM, noch bevorzugter 75 ± 15 mM, am Bevorzugtesten 75 ± 10 mM und insbesondere 75 ± 5 mM. In einer weiteren bevorzugten Ausführungsform ist die Gesamtkonzentration 100 ± 25 mM, bevorzugter 100 ± 20 mM, noch bevorzugter 100 ± 15 mM, am Bevorzugtesten 100 ± 10 mM und insbesondere 100 ± 5 mM. In einer weiteren bevorzugten Ausführungsform ist die Gesamtkonzentration 200 ± 25 mM, bevorzugter 200 ± 20 mM, noch bevorzugter 200 ± 15 mM, am Bevorzugtesten 200 ± 10 mM und insbesondere 200 ± 5 mM. Die Gesamtkonzentration ist vorzugsweise berechnet mittels des mittleren Molekulargewichts der erfindungsgemäßen carboxylierten Stärkederivate.

Die erfindungsgemäße Dialyselösung enthält erfindungsgemäße carboxylierte Stärkederivate in einer Gesamtmassenkonzentration von vorzugsweise 0,01 g/L bis 1,0 kg/L, bevorzugter von 0,1 bis 750 g/L, noch bevorzugter von 1,0 bis 500 g/L, am Bevorzugtesten 10 bis 250 g/L und insbesondere von 100 bis 200 g/L. In einer bevorzugten Ausführungsform ist die Gesamtmassenkonzentration 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

Die erfindungsgemäße Dialyselösung kann auch weitere osmotisch aktive Substanzen wie beispielsweise Glucose, Polyglucose, quervernetzte Glucose oder Polyglucose, Mannitol oder Glycerol enthalten.

Die erfindungsgemäße Dialyselösung enthält vorzugsweise einen oder mehrere Elektrolyte.

Im Sinne dieser Erfindung steht der Ausdruck "Elektrolyt" für eine Substanz, die freie Ionen enthält und elektrische Konduktivität aufweist. Vorzugsweise dissoziiert das Elektrolyt vollständig in Kationen und Anionen ohne den pH-Wert einer wässrigen Zusammensetzung im Wesentlichen zu ändern. Diese Eigenschaft grenzt Elektrolyte von Puffersubstanzen ab. Vorzugsweise liegen die Elektrolyte in einer Konzentration vor, die in einer im Wesentlichen vollständigen Dissoziation in Wasser resultiert.

Bevorzugte Elektrolyte sind ausgewählt aus der Gruppe der Alkalimetalle wie beispielsweise Na⁺ und K⁺ und der Erdalkalimetalle wie beispielsweise Ca²⁺ und Mg²⁺. Ein bevorzugtes Anion ist Cl-.

Die erfindungsgemäße Dialyselösung kann weitere Anionen wie beispielsweise Hydrogencarbonat, Dihydrogenphosphat, Hydrogenphosphat, Phosphat, Acetat, Lactat und Pyruvat enthalten; diese Anionen (in geeigneten Kombinationen mit Kationen) werden jedoch aufgrund Ihrer Pufferkapazität im Sinne dieser Erfindung nicht als Elektrolyte sondern als Puffer bezeichnet.

In einer bevorzugten Ausführungsform enthält die erfindungsgemäße Dialyselösung Na⁺-Ionen. Die Konzentration an Na⁺-Ionen ist vorzugsweise 10 bis 200 mM oder 50 bis 190 mM, bevorzugter 100 bis 180 mM oder 110 bis 170 mM, noch bevorzugter 115 bis 165 mM oder 120 bis 160 mM, am Bevorzugtesten 125 bis 155 mM und insbesondere 130 bis 150 mM. In einer weiteren bevorzugten Ausführungsform enthält die erfindungsgemäße Dialyselösung keine Na⁺-Ionen.

In einer bevorzugten Ausführungsform enthält die erfindungsgemäße Dialyselösung K⁺-Ionen. Die Konzentration an K⁺-Ionen ist vorzugsweise 0,10 bis 20 mM, bevorzugter 0,25 bis 15 mM, noch bevorzugter 0,50 bis 10 mM, am Bevorzugtesten 0,75 bis 7,5 mM und insbesondere 1,0 bis 5,0 mM. In einer weiteren bevorzugten Ausführungsform ist die Konzentration an K⁺-Ionen 1,0 ± 0,75, 2,0 ± 0,75, 3,0 ± 0,75, 4,0 ± 0,75 oder 5,0 ± 0,75 mM und insbesondere 1,0 ± 0,50, 2,0 ± 0,50, 3,0 ± 0,50, 4,0 ± 0,50 oder 5,0 ± 0,50. In einer weiteren bevorzugten Ausführungsform enthält die erfindungsgemäße Dialyselösung keine K⁺-Ionen.

In einer bevorzugten Ausführungsform enthält die erfindungsgemäße Dialyselösung Ca²⁺-Ionen. Die Konzentration an Ca²⁺-Ionen ist vorzugsweise 0,1 bis 3 mM, bevorzugter 0,25 bis 2,75 mM, noch bevorzugter 0,5 bis 2,5 mM, am Bevorzugtesten 0,75 bis 2,25 mM und insbesondere 1 bis 2 mM. In einer weiteren bevorzugten Ausführungsform ist die Konzentration an Ca²⁺-Ionen 0,25, 0,5, 0,75, 1, 1,25, 1,5, 1,75 oder 2 mM. In einer weiteren bevorzugten Ausführungsform enthält die erfindungsgemäße Dialyselösung keine Ca²⁺-Ionen.

In einer bevorzugten Ausführungsform enthält die erfindungsgemäße Dialyselösung Mg²⁺-Ionen. Die Konzentration an Mg²⁺-Ionen ist vorzugsweise 0,01 bis 1 mM, bevorzugter 0,05 bis 0,75 mM, noch bevorzugter 0,1 bis 0,5 mM, am Bevorzugtesten 0,15 bis 0,4 mM und insbesondere 0,2 bis 0,3 mM. In einer weiteren bevorzugten Ausführungsform ist die Konzentration an Mg²⁺-Ionen 0,05, 0,075, 0,1, 0,2, 0,25, 0,50 oder 0,75 mM. In einer weiteren bevorzugten Ausführungsform enthält die erfindungsgemäße Dialyselösung keine Mg²⁺-Ionen.

In einer bevorzugten Ausführungsform enthält die erfindungsgemäße Dialyselösung Cl⁻-Ionen. Die Konzentration an Cl⁻-Ionen ist vorzugsweise 10 bis 300 mM, bevorzugter 25 bis 250 mM, noch bevorzugter 50 bis 200 mM, am Bevorzugtesten 75 bis 150 mM und insbesondere 80 bis 125 mM. In einer weiteren bevorzugten Ausführungsform ist die Konzentration an Cl⁻-Ionen 100 ± 50 mM, bevorzugter 100 ± 25 mM, am Bevorzugtesten 100 ± 10 mM und insbesondere 96 ± 4 mM. In einer weiteren bevorzugten Ausführungsform enthält die erfindungsgemäße Dialyselösung keine Cl⁻-Ionen.

Die erfindungsgemäße Dialyselösung enthält vorzugsweise einen oder mehrere Puffer.

Geeignete Puffer sind dem Fachmann bekannt. Üblicherweise umfassen Puffer Lactat-, Hydrogencarbonat-, Carbonat-, Dihydrogenphosphat-, Hydrogenphosphat-, Phosphat-, Pyruvat-, Citrat-, Isocitrat-, Succinat-, Fumarat-, Acetat- und Lactat-Salze. Der Fachmann weiß, dass das entsprechende Kation der vorstehend genannten Anionen Bestandteil des Puffers ist, der zur Einstellung des pH-Wertes benutzt wird (z.B. Na⁺ als Bestandteil des Puffers NaHCO₃). Wenn das Puffersalz jedoch in Wasser dissoziiert hat es auch die Wirkung eines Elektrolyts. Für die Zwecke dieser Beschreibung berechnen sich die Konzentrationen an Kationen oder Anionen und die Gesamtkonzentration an Ionen, unabhängig davon, ob sie als Bestandteil von Elektrolyten, Puffern oder anderen Verbindungen (z.B. als Salze der erfindungsgemäßen carboxylierten Stärkederivate) eingesetzt werden.

In einer bevorzugten Ausführungsform enthält der Puffer Hydrogencarbonat. Hydrogencarbonat ist ein gut verträgliches Puffersystem, das im alkalischen Milieu mit Carbonat und im sauren Milieu mit H₂CO₃ bzw. CO₂ im Gleichgewicht steht. Neben Hydrogencarbonat sind auch andere Puffer-Systeme einsetzbar, die im Bereich von pH 4 bis pH 8, bevorzugter im Bereich von pH 5 bis pH 7,6 und insbesondere im Bereich von pH 7,6, 7,4, 7,2 und/oder 7,0 eine Pufferwirkung ausüben; z.B. auch Verbindungen, die im Körper zu Hydrogencarbonat metabolisiert werden können wie Lactat oder Pyruvat.

In einer weiteren bevorzugten Ausführungsform enthält der Puffer das Salz einer schwachen Säure, vorzugsweise Lactat. Die Säurestärke (pKs) der schwachen Säure ist vorzugsweise ≤5. Der Puffer kann auch ein Gemisch von Substanzen mit Pufferwirkung sein, z.B. ein Gemisch enthaltend Hydrogencarbonat und ein Salz einer schwachen Säure (z.B. Lactat). Eine geringe Hydrogencarbonat-Konzentration hat den Vorteil, dass der CO₂-Druck im Behältnis gering ist.

In einer bevorzugten Ausführungsform ist die erfindungsgemäße Dialyselösung gepuffert durch Hydrogencarbonat. Die Hydrogencarbonat-Konzentration ist vorzugsweise 1,0 bis 200 mM, bevorzugter 2,5 bis 150 mM, noch bevorzugter 5 bis 100 mM, am Bevorzugtesten 5 bis 75 mM oder 10 bis 50 mM und insbesondere 20 bis 30 mM. In einer weiteren bevorzugten Ausführungsform ist die Hydrogencarbonat-Konzentration 25 mM. In einer weiteren bevorzugten Ausführungsform enthält die erfindungsgemäße Dialyselösung kein Hydrogencarbonat.

In einer bevorzugten Ausführungsform ist die erfindungsgemäße Dialyselösung gepuffert durch Lactat. Die Lactat-Konzentration ist vorzugsweise 1,0 bis 200 mM, bevorzugter 2,5 bis 150 mM, noch bevorzugter 5 bis 100 mM, am Bevorzugtesten 10 bis 50 mM oder 10 bis 25 mM und insbesondere 15 mM. In einer weiteren bevorzugten Ausführungsform enthält die erfindungsgemäße Dialyselösung kein Lactat.

In einer bevorzugten Ausführungsform ist die erfindungsgemäße Dialyselösung gepuffert durch Acetat. Die Acetat-Konzentration ist vorzugsweise 1,0 bis 100 mM, bevorzugter 1,0 bis 50 mM, noch bevorzugter 1,0 bis 25 mM, am Bevorzugtesten 1,0 bis 10 mM oder 2,0 bis 7,5 mM und insbesondere 2,5 bis 7,0 mM. In einer weiteren bevorzugten Ausführungsform enthält die erfindungsgemäße Dialyselösung kein Acetat.

Das Gesamtvolumen an Dialyselösung ist nicht beschränkt. Üblicherweise beträgt das Volumen mehrere Liter (geeignetes Verabreichungsvolumen für einen Patienten) bis einige hundert Liter (geeignetes Vorratsvolumen für mehr als einen Patienten).

Wie bereits oben stehend ausgeführt ist unter dem Ausdruck "Dialyselösung" im Sinne dieser Erfindung eine gebrauchsfertige Dialyselösung zu verstehen, d.h. die Dialyselösung kann direkt für die Dialysebehandlung (Hämodialyse oder Peritonealdialyse) verwendet werden.

In einer bevorzugten Ausführungsform ist die erfindungsgemäße Dialyselösung eine Peritonealdialyselösung wie nachstehend beschrieben.

Die Peritonealdialyselösung ist biochemisch so abgestimmt, dass sie die mit Nierenversagen einhergehende metabolische Azidose im Wesentlichen korrigiert. Die Peritonealdialyselösung enthält Hydrogencarbonat vorzugsweise in annähernd physiologischen Konzentrationen. In einer bevorzugten Ausführungsform enthält die Peritonealdialyselösung Hydrogencarbonat in einer Konzentration von ca. 20 bis 30 mM. In einer weiteren bevorzugten Ausführungsform enthält die Peritonealdialyselösung eine Hydrogencarbonat-Konzentration von 25 mM.

Ferner enthält die Peritonealdialyselösung vorzugsweise Kohlenstoffdioxid mit einem Partialdruck (p_{CO2}) von weniger als 60 mmHg. In einer bevorzugten Ausführungsform ist p_{CO2} der Peritonealdialyselösung im Wesentlichen gleich zum p_{CO2}, der in Blutgefäßen gemessen wird.

Ferner hat die Peritonealdialyselösung vorzugsweise einen pH-Wert von ca. 7,4. Daher ist die Peritonealdialyselösung eine physiologisch verträgliche Lösung.

Die Peritonealdialyselösung enthält vorzugsweise eine schwache Säure mit einem pKs ≤5. Die schwachen Säuren sind vorzugsweise Verbindungen, die als physiologische Stoffwechselprodukte im Glucose-Metabolismus auftreten. Die schwache Säure ist vorzugsweise ausgewählt aus Gruppe bestehend aus Lactat, Pyruvat, Citrat, Isocitrat, Ketoglutarat, Succinat, Fumarat, Malat und Oxaloacetat. Diese Säuren können entweder allein oder als Gemisch in der Peritonealdialyselösung enthalten sein. Die schwachen Säuren sind vorzugsweise in einer Konzentration von 10 bis 20 mEq/L und im Wesentlichen als Natrium-Salze in der Peritonealdialyselösung enthalten. In der Peritonealdialyselösung ist die schwache Säure vorzugsweise in einer Menge enthalten, die der täglichen metabolischen Wasserstoffproduktion von ca. 1 mEq/kg/Tag entspricht.

Die Peritonealdialyselösung enthält wenigstens eine erfindungsgemäße carboxylierte Stärke oder ein erfindungsgemäßes carboxyliertes Stärkederivat wie oben stehend definiert.

Die erfindungsgemäße Peritonealdialyselösung enthält vorzugsweise eine Konzentration an Hydrogencarbonat und weist einen p_{CO2} auf, wie sie bei gesunden, nicht-niereninsuffizienten Patienten gemessen werden. Die schwache Säure diffundiert entlang des Konzentrationsgradienten von der Dialyselösung ins Blut des Dialysepatienten und korrigiert somit die metabolische Azidose der Dialysepatienten.

Ein weiterer Gegenstand dieser Erfindung betrifft Mehrkomponenten-Systeme zur Herstellung der oben beschriebenen gebrauchsfertigen Dialyselösungen. Die Herstellung erfolgt vorzugsweise in einer detailliert beschriebenen Art und Weise, d.h. durch Befolgung einer entsprechenden Anleitung (Protokoll). Besagte Herstellung kann manuell, z.B. durch Mischen einzelner Komponenten oder Verdünnen einer Komponente mit Wasser, erfolgen. Die Herstellung kann jedoch auch automatisiert erfolgen, z.B. mittels einer Vorrichtung die für diesen Prozess geeignet ist und kommerziell erhältlich sein kann. Die Herrichtung muss nicht zwangsläufig zu einer Dialyselösung mit statischer (gleich bleibender) Zusammensetzung führen sondern kann auch zu einer Dialyselösung führen, die kontinuierlich ihre Zusammensetzung ändert, wobei diese Änderung durch eine geeignete Vorrichtung überwacht werden kann. Beispielsweise kann die erfindungsgemäß carboxylierte Stärke in einer Dialyselösung enthalten sein, die kontinuierlich während der Dialysebehandlung verdünnt wird, so dass der Patient einer abnehmenden Konzentration von carboxylierter Stärke ausgesetzt ist.

In einer bevorzugten Ausführungsform sind die erfindungsgemäßen Dialyselösungen zur Verwendung in der Behandlung der Niereninsuffizienz geeignet.

In einer weiteren bevorzugten Ausführungsform sind die erfindungsgemäßen Dialyselösungen zur Verwendung in der Dialysebehandlung geeignet.

In einer weiteren bevorzugten Ausführungsform sind die erfindungsgemäßen Dialyselösungen zur Verwendung in der Hämodialyse- und/oder Peritonealdialysebehandlung geeignet.

Ein weiterer Gegenstand dieser Erfindung betrifft ein Kit, das für die Herstellung der erfindungsgemäßen Dialyselösungen konfiguriert ist, wobei das Kit
- eine erste Komponente, die eine sterile Lösung ist und eine Säure enthält und einen pH-Wert ≤6,0 aufweist,
- eine zweite Komponente die eine sterile Lösung ist und eine Säure enthält und einen pH-Wert ≥7,0 aufweist und
- ggf. eine weitere oder mehrere weitere Komponenten umfasst,
wobei mindestens eine der beiden Komponenten ein erfindungsgemäßes carboxyliertes Stärkederivat enthält und
die Herstellung der erfindungsgemäßen Dialyselösung durch Mischen der ersten Komponente mit der zweiten Komponente und ggf. der/ den weiteren Komponente(n) erfolgt.

Das Kit umfasst wenigstens eine erste Komponente und eine zweite Komponente. Das Kit kann auch weitere Komponenten umfassen, z.B. eine dritte und eine vierte Komponente. Vorzugsweise besteht das Kit aus zwei Komponenten, die vorzugsweise voneinander verschieden sind.

Im Sinne dieser Erfindung umfasst der Ausdruck "Komponente" flüssige, halbfeste oder feste Zusammensetzungen, die zueinander gleich oder voneinander verschieden sein können, wobei durch Mischen aller Komponenten des Kits die erfindungsgemäße gebrauchsfertige Dialyselösung erhalten wird. Vorzugsweise enthält eine einzelne Komponente einen Teil der Inhaltsstoffe, die in der gebrauchsfertigen Dialyselösung enthalten sind.

Die erste und die zweite Komponente können, unabhängig voneinander, fest, halbfest oder flüssig sein. Im Falle, dass die Komponenten flüssig sind, können sie Lösungen oder Dispersionen (z.B. Dispersionen oder Suspensionen) sein.

In einer bevorzugten Ausführungsform ist die erste Komponente flüssig, vorzugsweise reines Wasser oder eine wässrige Lösung, und die zweite Komponente ist ebenfalls flüssig. In einer weiteren bevorzugten Ausführungsform ist die erste Komponente flüssig, vorzugsweise reines Wasser oder eine wässrige Lösung, und die zweite Komponente ist fest, vorzugsweise ein pulverförmiges Gemisch.

Die erste Komponente ist vorzugsweise eine Lösung, die osmotisch wirksame Substanzen (z.B. erfindungsgemäße carboxylierte Stärkederivate), Calcium-Ionen, Magnesium-Ionen, Hydronium-Ionen und Chlorid-Ionen enthält.

Das erfindungsgemäße Kit kann verschiedenartig ausgestaltet sein. Beispielsweise können die einzelnen Komponenten in voneinander separierten Behältnissen (z.B. einzelne Beutel) vorliegen. Das erfindungsgemäße Kit ist jedoch vorzugsweise ein Gebinde, wie beispielsweise ein Mehrkammer-Behältnis-System (z.B. flexibles oder starres Mehrkammer-Behältnis-System), vorzugsweise ein flexibles Mehrkammer-Beutel-System.

Das erfindungsgemäße Kit ist vorzugsweise ein Mehrkammer-Behältnis-System, das die erste Komponente, die zweite Komponente und ggf. eine oder mehrere weitere Komponenten in Kammern enthält, die voneinander durch lösbare bzw. brechbare Trennsysteme (z.B. Trennbrechteile) getrennt sind, wobei die erste Komponente, die zweite Komponente und ggf. die eine oder mehreren weiteren Komponenten nach dem Lösen bzw. Brechen des Trennsystems miteinander gemischt werden können, um die erfindungsgemäße Dialyselösung zu erhalten.

Das Mehrkammer-Behältnis kann als Kunststoffgebinde vorliegen (z.B. Mehrkammer-Kunststoffbeutel), der jeweils eine abgetrennte Kammer für jede einzelne Komponente enthält. Vorzugsweise enthält das Kunststoffgebinde die einzelnen Komponentenlösungen in Kammern, die jeweils durch Trennelemente voneinander abgetrennt sind.

Das Mehrkammer-Behältnis ist vorzugsweise ein Zweikammer-Beutel umfassend ein Kunststoffgebinde mit einer ersten Kammer und einer zweiten Kammer, wobei die Kammern durch ein lösbares bzw. brechbares Trennsystem voneinander getrennt sind, und die erste Kammer die erste Komponente enthält und die zweite Kammer die zweite Komponente enthält. Das Lösen bzw. Brechen des Trennsystems führt zum Mischen der beiden Komponenten und resultiert in der gebrauchsfertigen Dialyselösung. Die erste Kammer und die zweite Kammer sind im Gebinde vorzugsweise angrenzend angeordnet und durch das Trennsystem voneinander abgetrennt. Das Trennsystem ist vorzugsweise eine Trennnaht (z.B. lösbare oder brechbare Schweißnaht). Die Trennnaht öffnet sich vorzugsweise durch das Anlegen eines Druckes auf eine der Kammern, woraufhin die Trennnaht bricht bzw. sich löst und sich der Inhalt der beiden Kammern mischt und das Gemisch als gebrauchsfertige Dialyselösung in der Dialysebehandlung eingesetzt werden kann.

Die erste Komponente des erfindungsgemäßen Kits ist eine sterile Lösung, die eine Säure enthält und einen pH-Wert ≤ 6,0 aufweist; die zweite Komponente ist ebenfalls eine sterile Lösung, die einen Puffer enthält und einen pH-Wert ≥ 7,0 aufweist.

Die erfindungsgemäßen carboxylierten Stärkederivate können in der ersten Komponente oder in der zweiten Komponente als auch in beiden Komponenten in gleichen oder verschiedenen Konzentrationen enthalten sein. In einer bevorzugten Ausführungsform ist die erfindungsgemäße carboxylierte Stärke nur in der ersten (sauren) Komponente enthalten. In einer weiteren bevorzugten Ausführungsform sind die erfindungsgemäßen carboxylierten Stärkederivate nur in der zweiten (basischen) Komponente enthalten. Die erste Komponente und/ oder die zweite Komponente und/ oder die ggf. weitere(n) Komponente(n) können einen oder mehrere Elektrolyte aber auch Puffer enthalten.

Der Fachmann erkennt, dass das Mischen der einzelnen Komponenten üblicherweise einen Verdünnungseffekt für den Fall nach sich zieht, dass die Komponenten die Inhaltsstoffe in unterschiedlichen Konzentrationen enthalten. Falls beispielsweise die erfindungsgemäßen carboxylierten Stärkederivate ausschließlich in einer der Komponenten enthalten sind, führt das Mischen dieser Komponente mit wenigstens einer anderen Komponente zu einem Anstieg des Volumens in Bezug auf die vorhandene Menge der erfindungsgemäßen carboxylierten Stärkederivate und somit zu einer Verdünnung, d.h. Abnahme der Konzentration von carboxylierten Stärkederivaten; folglich enthält die Komponente die erfindungsgemäßen carboxylierten Stärkederivate vorzugsweise in einer höheren Konzentration als die gebrauchsfertige Dialyselösung.

Vorzugsweise ist die Konzentration an erfindungsgemäßer carboxylierter Stärke oder erfindungsgemäßen carboxylierten Stärkederivaten in der Komponente nahe an der Sättigungskonzentration bei einer Temperatur von 5 °C um eine ausreichende Lagerstabilität bei höheren Temperaturen sicher zu stellen.

In einer bevorzugten Ausführungsform ist die Gesamtmassenkonzentration an erfindungsgemäßer carboxylierter Stärke oder Stärkederivaten in der Komponente 0,01 g/L bis 1,0 kg/L, bevorzugter 0,1 bis 750 g/L, noch bevorzugter 1,0 bis 500 g/L, am Bevorzugtesten 10 bis 250 g/L und insbesondere 100 bis 200 g/L. In einer weiteren bevorzugten Ausführungsform ist die Gesamtmassenkonzentration an erfindungsgemäßer carboxylierter Stärke oder Stärkederivaten in der Komponente 25 ± 24 g/L, bevorzugter 25 ± 20 g/L, noch bevorzugter 25 ± 15 g/L, am Bevorzugtesten 25 ± 10 g/L und insbesondere 25 ± 5 g/L. In einer weiteren bevorzugten Ausführungsform ist die Gesamtmassenkonzentration an erfindungsgemäßer carboxylierter Stärke oder Stärkederivaten in der Komponente 50 ± 25 g/L, bevorzugter 50 ± 20 g/L, noch bevorzugter 50 ± 15 g/L, am Bevorzugtesten 50 ± 10 g/L und insbesondere 50 ± 5 g/L. In einer weiteren bevorzugten Ausführungsform ist die Gesamtmassenkonzentration an erfindungsgemäßer carboxylierter Stärke oder Stärkederivaten in der Komponente 75 ± 25 g/L, bevorzugter 75 ± 20 g/L, noch bevorzugter 75 ± 15 g/L, am Bevorzugtesten 75 ± 10 g/L und insbesondere 75 ± 5 g/L. In einer weiteren bevorzugten Ausführungsform ist die Gesamtmassenkonzentration an erfindungsgemäßer carboxylierter Stärke oder Stärkederivaten in der Komponente 100 ± 25 g/L, bevorzugter 100 ± 20 g/L, noch bevorzugter 100 ± 15 g/L, am Bevorzugtesten 100 ± 10 g/L und insbesondere 100 ± 5 g/L. In einer weiteren bevorzugten Ausführungsform ist die Gesamtmassenkonzentration an erfindungsgemäßer carboxylierter Stärke oder Stärkederivaten in der Komponente 200 ± 25 g/L, bevorzugter 200 ± 20 g/L, noch bevorzugter 200 ± 15 g/L, am Bevorzugtesten 200 ± 10 g/L und insbesondere 200 ± 5 g/L.

In einer bevorzugten Ausführungsform enthält die zweite Komponente die Gesamtmenge an erfindungsgemäßer carboxylierter Stärke oder erfindungsgemäßen carboxylierten Stärkederivaten und einen geeigneten Puffer, der den pH-Wert der zweiten Komponente auf über 7,0, bevorzugter auf über 7,5, noch bevorzugter auf über 8,0, am Bevorzugtesten auf über 8,5 und insbesondere auf über 9,0 einstellt. Dies kann vorzugsweise durch Hydrogencarbonat erreicht werden, die beispielsweise in Form von dissoziiertem Natrium-Hydrogencarbonat und oder Kalium-Hydrogencarbonat vorliegen können. In einer weiteren bevorzugten Ausführungsform ist die zweite Komponente fest und umfasst ein pulverförmiges Gemisch enthaltend wenigstens eine erfindungsgemäße carboxylierte Stärke oder wenigstens ein erfindungsgemäßes carboxyliertes Stärkederivat und wenigstens einen Puffer, z.B. Natrium- und/ oder Kalium-Hydrogencarbonat.

Der Mehrkammer-Beutel ist vorzugsweise geeignet für die Herstellung einer Dialyselösung, die zur Verwendung in der Peritonealdialysebehandlung verwendet werden kann, und die folgenden Inhaltsstoffe vorzugsweise in folgenden Konzentrationen enthält:
Ca²⁺ 0,5 bis 5 mval/L;
Mg²⁺ 0 bis 3,0 mval/L;
Cl⁻ 90,5 bis 121 mval/L;
K⁺ 0 bis 4,0 mval/L;
HCO₃⁻ 25 bis 40 mval/L; wobei
eine Kammer des Mehrkammer-Beutel-Systems ein erstes saures Konzentrat und eine andere Kammer ein zweites basisches Konzentrat enthält; wobei das saure Konzentrat Ca²⁺-Ionen enthält und das basische Konzentrat HCO₃⁻-Ionen aber keine Ca²⁺-Ionen enthält; und die zwei Konzentrate nach Lösen bzw. Brechen des Trennsystems (z.B. Trennnaht) miteinander gemischt werden können; wobei das Mischen der zwei Konzentrate zur Herstellung der gebrauchsfertigen Dialyselösung führt und der pH der gebrauchsfertigen Dialyselösung 7,0 bis 7,6 ist.

Vorzugsweise enthält das basische Konzentrat wenigstens eine erfindungsgemäße carboxylierte Stärke oder wenigstens ein erfindungsgemäßes carboxyliertes Stärkederivat und ggf. Glucose und/ oder Polyglucose, wohingegen das saure Konzentrat keine erfindungsgemäße carboxylierte Stärke oder Stärkederivat und keine Glucose und/ oder Polyglucose enthält.

Vorzugsweise enthält das basische Konzentrat eine Menge an Hydrogencarbonat, die zu einer Hydrogencarbonat-Konzentration der gebrauchsfertigen Dialyselösung von wenigstens 20 mM führt. Vorzugsweise ist die Hydrogencarbonat-Konzentration der basischen Komponente so hoch, dass die gebrauchsfertige Dialyselösung eine Hydrogencarbonat-Konzentration von 25 mM aufweist.

Der pH-Wert des basischen, gepufferten zweiten Konzentrats wird vorzugsweise mit Salzsäure eingestellt.

Vorzugsweise werden die beiden Konzentrate in einem Volumenverhältnis von 10:1 bis 1:10 oder 8:1 bis 1:8, bevorzugter 5:1 bis 1:5 oder 3:1 bis 1:3, noch bevorzugter 2:1 bis 1:2 und insbesondere 1:1 miteinander gemischt.

Der Mehrkammer-Beutel weist vorzugsweise eine Gasbarriere-Folie auf, die verhindert, das gasförmiges CO₂ aus dem System entweicht. Gasbarriere-Folien sind dem Fachmann bekannt.

Ein bevorzugter Gegenstand dieser Erfindung betrifft ein Verfahren zur Herstellung einer Dialyselösung, worin das gewünschte Mischverhältnis automatisch durch eine Dialysemaschine oder einen Peritonealdialyse-Cycler erfolgt.

Ein weiterer Gegenstand dieser Erfindung betrifft die Verwendung wenigstens einer erfindungsgemäßen carboxylierten Stärke oder wenigstens eines erfindungsgemäßen carboxylierten Stärkederivats zur Herstellung der erfindungsgemäßen Dialyselösung (Hämodialyselösung oder Peritonealdialyselösung).

Ein weiterer Gegenstand dieser Erfindung betrifft die Verwendung eines erfindungsgemäßen Kits zur Herstellung der erfindungsgemäßen Dialyselösung (Hämodialyselösung oder Peritonealdialyselösung).

### Beispiele

Die Verbindungen der Beispiele 1 und 2 wurden in Anlehnung an eine Vorschrift von T. Heinze *et al.* hergestellt (T. Heinze, M. Vieira, U. Heinze; New polymers based on cellulose; Lenzinger Berichte 79 (2000) 39-44). Die Analytik der Proben erfolgte mittels Gelpermeationschromatographie (GPC), ¹³C NMR- und FTIR-Spektroskopie, Elementaranalyse und konduktometrische Titration. Das verwendete Icodextrin hat laut GPC-Messungen eine Molmasse von ca. 5800 g/mol (bezogen auf das Zahlenmittel, Mn) bzw. ca. 16000 g/mol (bezogen auf das Massenmittel, Mw).

### Beispiel 1

In 3 I destilliertes Wasser wurden nacheinander 50 g (0,309 mol) Icodextrin, 0,41 g TEMPO (2,2,6,6-Tetramethyl-1-piperidinyloxylradikal, 2,63*10-3mol, entspricht: 8,5*10-3 mol / mol AGU) und 2,86 g NaBr (0,02781 mol, entspricht: 0,09 mol/mol AGU) eingerührt. Die Mischung wurde mit einem Eisbad auf ca. 1 °C abgekühlt. Die Mischung hatte einen pH von 5. Anschließend wurden 85 ml einer 10 Gew.-%ige NaOCl-Lösung unter Rühren langsam zugetropft. Dabei wurde wie folgt vorgegangen. Zu Beginn wurde soviel Lösung zugetropft bis ein pH von 10,8 erreicht war. Der pH-Wert der Mischung wurde weiter überwacht. Sobald der pH unter 10,8 sank wurde weitere NaOCl-Lösung langsam zugetropft. Nach einer Stunde wurde eine klare Lösung erhalten. Die carboxylierte Stärke wurde durch Einrühren der Lösung in 10 l Ethanol ausgefällt, abfiltriert, mit Ethanol/Wasser (v/v = 8/2) nachgewaschen und im Vakuum bei 50 °C getrocknet.
Ausbeute: 50 g;
Oxidationsgrad (DO, über konduktometrische Titration bestimmt): 0,21

### Beispiel 2

In 3 l destilliertes Wasser wurden nacheinander 50 g (0,309 mol) Icodextrin, 0,41 g TEMPO (2,2,6,6-Tetramethyl-1-piperidinyloxylradikal, 2,63*10-3mio!, entspricht: 8,5*10-3 mol / mol AGU) und 2,86 g NaBr (0,02781 mol, entspricht: 0,09 mol / mol AGU) eingerührt. Die Mischung wurde mit einem Eisbad auf ca. 1 °C abgekühlt. Die Mischung hatte einen pH von 5. Anschließend wurden 640 ml einer 10 Gew.-%ige NaOCl-Lösung unter Rühren langsam zugetropft. Dabei wurde wie folgt vorgegangen. Zu Beginn wurde soviel Lösung zugetropft bis ein pH von 10,8 erreicht war. Der pH-Wert der Mischung wurde weiter überwacht. Sobald der pH unter 10,8 sank wurde weitere NaOCl-Lösung langsam zugetropft. Nach einer Stunde wurde eine klare Lösung erhalten. Die carboxylierte Stärke wurde durch Einrühren der Lösung in 10 l Ethanol ausgefällt, abfiltriert, mit Ethanol/Wasser (v/v = 8/2) nachgewaschen und im Vakuum bei 50 °C getrocknet.
Ausbeute: 50 g;
Oxidationsgrad (DO, über konduktometrische Titration bestimmt): 0,83

### Beispiel 3

In einem Vergleichsversuch wurde die osmotische Aktivität der carboxylierten Stärkederivate aus Beispiel 1 und 2, von unbehandeltem Icodextrin, sowie von Glukose untersucht.

Für den vergleichenden Versuch wurde ein Füllvolumen von 10 ml eines osmotischen Agens in einer Konzentration von 5 % (m/m) in einer Versuchslösung mit 1 mmol/l Ca²⁺, 0,5 mmol/l Mg²⁺, 138 mmol/l Na⁺, 106 mmol/l Cl⁻ und 35 mmol/l Lactat in einen semipermeablen Schlauch (regenerierte Cellulose, Molecular Weight Cut Off (MWCO): 1000 Dalton, Fa. Roth) gefüllt und bei einer Temperatur von 38°C in einem Bad der gleichen Versuchslösung für 24 Stunden unter Bewegung gelagert. Zu verschiedenen Zeitpunkten wurde die Volumenzunahme des Füllvolumens des Schlauchs ermittelt, die die osmotische Wirkung des Agens widerspiegelt.

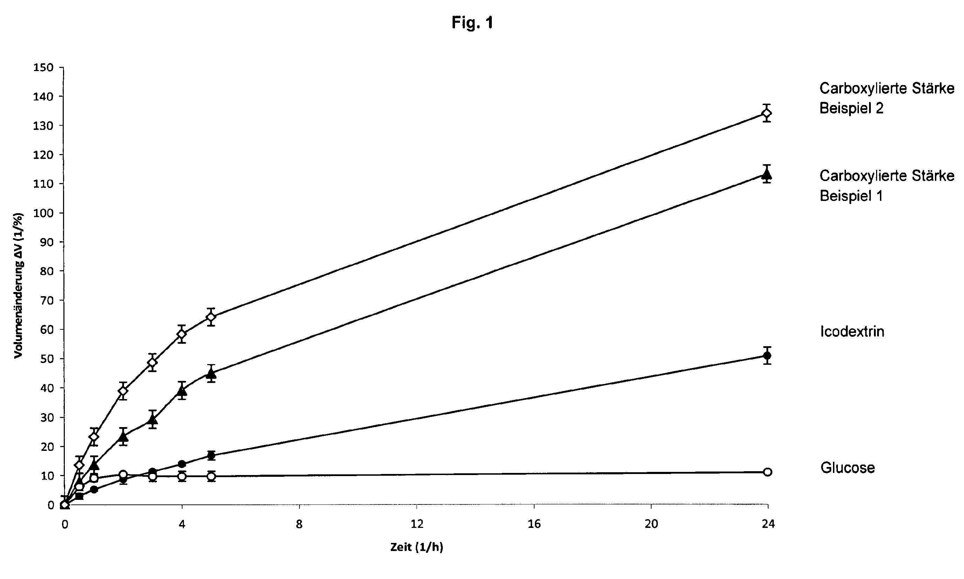

Die Ergebnisse des Vergleichsversuchs sind in der Figur 1 als Diagramm dargestellt.

Bei den carboxylierten Stärken nach den Beispielen 1 und 2 betrug die Volumenzunahme nach 24 h 113% bzw. 134%, während sie bei Icodextrin 51% und bei Glukose nur 11% betrug. Die carboxylierten Stärken nach den Beispielen 1 und 2 zeigen damit gegenüber Glukose und Icodextrin eine stärkere osmotische Wirkung.

## Patentansprüche

1. Carboxylierte Stärkederivate der allgemeinen Struktur I 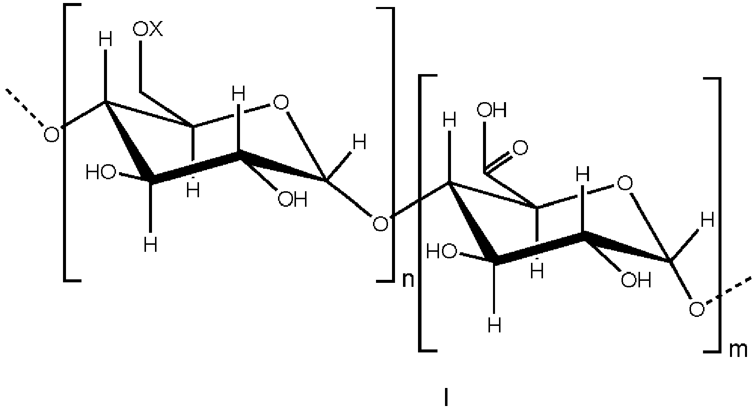 wobei
X entweder H oder eine Glucoseeinheit ist,
n und m Ganzzahlen und grösser oder gleich 1 sind und
der Oxidationsgrad bezogen auf den Rest -COOH zwischen ≥0,0001 und ≤1 beträgt, zur Verwendung in der Behandlung von chronischem Nierenversagen mittels Dialyse.

2. Carboxylierte Stärkederivate nach Anspruch 1 zur Verwendung nach Anspruch 1, wobei der Oxidationsgrad zwischen >0,01 und <1 beträgt.

3. Carboxylierte Stärkederivate nach Anspruch 1 oder 2 zur Verwendung nach Anspruch 1, wobei der Oxidationsgrad zwischen 0,05 und 0,98 beträgt.

4. Carboxylierte Stärkederivate nach einem oder mehreren der Ansprüche 1 bis 3 zur Verwendung nach Anspruch 1, wobei eine 7,5 gewichtsprozentige wässrige Lösung der carboxylierten Stärke eine Osmolarität >3 mosm/L aufweist.

5. Carboxylierte Stärkederivate nach einem oder mehreren der Ansprüche 1 bis 4 zur Verwendung in der Behandlung von chronischem Nierenversagen mittels Hämodialyse oder Peritonealdialyse.

6. Dialyselösung enthaltend wenigstens ein carboxyliertes Stärkederivat nach einem oder mehreren der Ansprüche 1 bis 4.

7. Kit, das für die Herstellung der Dialyselösung nach Anspruch 6 konfiguriert ist, umfassend
eine erste Komponente, die eine sterile Lösung ist und eine Säure enthält und einen pH-Wert ≤6,0 aufweist,
eine zweite Komponente, die eine sterile Lösung ist und einen Puffer enthält und einen pH-Wert ≥7,0 und
ggf. eine oder mehrere weitere Komponenten,
wobei mindestens eine der beiden Komponenten ein carboxyliertes Stärkederivat nach einem der oder mehreren der Ansprüche 1 bis 4 enthält, wobei durch Mischen der ersten Komponente mit der zweiten Komponente und ggf. mit der weiteren oder den weiteren Komponenten, die Dialyselösung nach Anspruch 6 erhalten wird.

8. Verwendung wenigstens eines carboxylierten Stärkederivats nach einem oder mehreren der Ansprüche 1 bis 5 oder
eines Kits nach Anspruch 7
zur Herstellung der Dialyselösung nach Anspruch 6.

## Claims

1. Carboxylated starch derivatives of general structure I 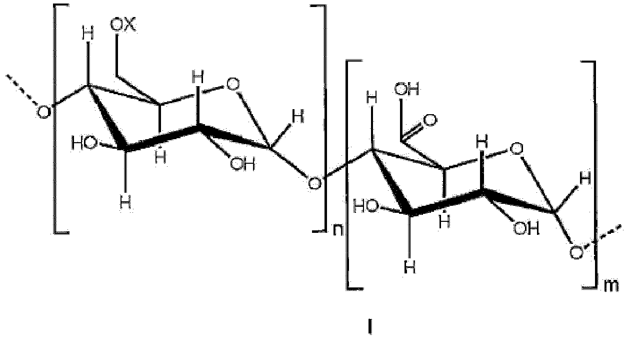 where
X is either H or a glucose unit,
n and m are integers greater than or equal to 1 and
the degree of oxidation, based on the -COOH radical, is between ≥0.0001 and ≤1, for use in the treatment of chronic renal failure by dialysis.

2. The carboxylated starch derivatives according to claim 1, for use according to claim 1, wherein the degree of oxidation is between >0.01 and <1.

3. The carboxylated starch derivatives according to claim 1 or 2, for use according to claim 1, wherein the degree of oxidation is between 0.05 and 0.98.

4. The carboxylated starch derivatives according to any one or more of claims 1 to 3, for use according to claim 1, wherein a 7.5% by weight aqueous solution of the carboxylated starch has an osmolarity >3 mosm/L.

5. The carboxylated starch derivatives according to any one or more of claims 1 to 4, for use in the treatment of chronic renal failure by hemodialysis or peritoneal dialysis.

6. A dialysis solution containing at least one carboxylated starch derivative according to any one or more of claims 1 to 4.

7. A kit, configured for preparation of the dialysis solution according to claim 6, comprising
a first component which is a sterile solution containing an acid and having a pH ≤6.0,
a second component, which is a sterile solution containing a buffer and having a pH ≥7.0, and
optionally one or more additional components,
wherein at least one of the two components contains a carboxylated starch derivative according to any one or more of claims 1 to 4,
wherein the dialysis solution according to claim 6 is obtained by mixing the first component with the second component and optionally with the additional component(s).

8. Use of at least one carboxylated starch derivative according to any one or more of claims 1 to 5 or
a kit according to claim 7,
for preparation of the dialysis solution according to claim 6.

## Revendications

1. Dérivé d'amidon carboxylé de structure générale I 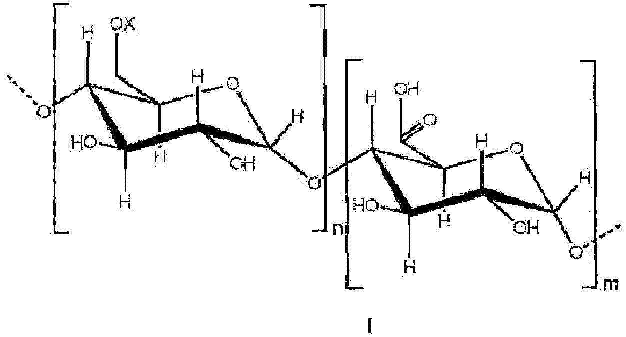 dans lequel
X est soit H soit une unité de glucose,
n et m sont des nombres entiers et sont supérieurs ou égaux à 1 et
le degré d'oxydation par rapport au reste de -COOH se situe entre ≥ 0,0001 et ≤ 1, destiné à un usage dans le traitement des déficiences rénales chroniques par dialyse.

2. Dérivé d'amidon carboxylé selon la revendication 1 à utiliser selon la revendication 1, le degré d'oxydation étant situé entre > 0,01 et < 1.

3. Dérivé d'amidon carboxylé selon la revendication 1 ou 2 à utiliser selon la revendication 1, le degré d'oxydation étant situé entre 0,05 et 0,98.

4. Dérivé d'amidon carboxylé selon une ou plusieurs des revendications 1 à 3 à utiliser selon la revendication 1, dans lequel une solution aqueuse à 7,5 % en poids d'amidon carboxylé présente une osmolarité > 3 mosm/L.

5. Dérivé d'amidon carboxylé selon une ou plusieurs des revendications 1 à 4, destiné à une utilisation dans le traitement des déficiences rénales chroniques par hémodialyse ou dialyse péritonéale.

6. Solution de dialyse contenant au moins un dérivé d'amidon carboxylé selon une ou plusieurs des revendications 1 à 4.

7. Kit, qui est configuré pour la préparation de la solution de dialyse selon la revendication 6, comprenant
un premier composant qui est une solution stérile et contient un acide et présente une valeur de Ph ≤ 6,0,
un second composant qui est une solution stérile et contient un tampon et présente une valeur de ph ≥ 7,0 et
le cas échéant un ou plusieurs autres composants,
au moins un des deux composants contenant un dérivé d'amidon carboxylé selon une ou plusieurs des revendications 1 à 4,
la solution de dialyse selon la revendication 6 étant obtenue par mélange du premier composant avec le second composant et le cas échéant avec l'autre ou les autres composants.

8. Utilisation d'au moins un dérivé d'amidon carboxylé selon une ou plusieurs des revendications 1 à 5 ou d'un kit selon la revendication 7
pour la préparation de la solution de dialyse selon la revendication 6.
